# EUROPEAN PATENT APPLICATION

(11) **EP 4 706 579 A1**
(43) Date of publication of application: **11.03.2026**
(21) Application number: 23936270.0
(22) Date of filing: 30.06.2023
(51) Int. Cl.: A61B 34/32, A61M 25/09, A61M 39/22, A61M 5/00

(54) **DEVICE FOR AUTOMATICALLY THREADING GUIDE WIRE INTO VALVE AND FULLY-AUTOMATIC RADIOGRAPHY ROBOT**

(30) Priority: 05.05.2023 CN 202310497840
(71) Applicant: Shenzhen Institute of Advanced Biomedical Robot Co., Ltd., Shenzhen, Guangdong 518000 (CN)
(72) Inventor: YAO, Gang, Shenzhen, Guangdong 518000 (CN); YANG, Weinan, Shenzhen, Guangdong 518000 (CN); LI, Jian, Shenzhen, Guangdong 518000 (CN)
(74) Representative: Torggler & Hofmann Patentanwälte - Innsbruck
(86) International application number: PCT/CN2023/105117
(87) International publication number: WO 2024/229962

(57) **Abstract**

A device for automatically threading a guide wire into a valve and a fully-automatic radiography robot. Said device comprises a controller, and a catheter driving mechanism (1), a guide wire driving mechanism (2) and a threading assistant assembly (3) which are in communication connection to the controller. The catheter driving mechanism (1) is provided with a radiography valve body (11), the radiography valve body (11) being connected to a catheter (12) on the catheter driving mechanism (1). The guide wire driving mechanism (2) comprises a guide wire moving mechanism (21) and a guide wire delivery mechanism (211) mounted on the guide wire moving mechanism (21). The threading assistant assembly (3) is mounted on the side of the guide wire driving mechanism (2) close to the catheter driving mechanism (1). In a working state, a guide wire is clamped by the guide wire driving mechanism (2) and partially enters the threading assistant assembly (3); then the controller acquires a wire threading instruction, and, on this basis, controls the guide wire moving mechanism (21) to move towards the catheter driving mechanism (1) until part of the threading assistant assembly (3) enters the radiography valve body (11); and then controls the guide wire delivery mechanism (211) to deliver the guide wire into the catheter (12). The present application does not need doctors to perform manual operation on guide wires.

## Description

The present application claims the priority to Chinese Patent Application No. 202310497840.2, titled "DEVICE FOR AUTOMATICALLY THREADING GUIDE WIRE INTO VALVE AND FULLY-AUTOMATIC RADIOGRAPHY ROBOT", filed on May 5, 2023 with the China National Intellectual Property Administration, which is incorporated herein by reference in its entirety.

### FIELD

The present application relates to the technical field of medical robots, and in particular to a device for automatically threading a guidewire into a valve and a fully-automatic angiographic robot.

### BACKGROUND

The guidewire of angiographic robots used in vascular intervention is typically quite long, which results in the guidewire being exposed outside during the angiographic procedure. This poses risks of the guidewire falling or coming into contact with contaminated areas, increasing safety hazards during the procedure. Moreover, in current angiographic procedures, operators often need to manually monitor the status of the guidewire throughout the entire process, preventing them from fully focusing on the procedure itself. What's more, when the contrast agent is injected, the guidewire needs to be withdrawn from the valve body. When another angiography is needed, the operators have to reinsert the guidewire into the valve body again, significantly reducing the procedural efficiency. Additionally, during the angiographic procedure, when the guidewire is withdrawn from the human body and exposed outside, blood tends to adhere to its surface and clots on the guidewire, which leads to thrombus formation, further increasing the safety risks during the procedure.

### SUMMARY

### TECHNICAL ISSUE

A main object of the present application is to provide a device for automatically threading a guidewire into a valve and a fully-automatic angiographic robot, aiming to address the technical issue of improving the angiographic robot and its device for automatically threading the guidewire into the valve to make angiographic procedures more efficient and safer.

### TECHNICAL SOLUTION

A device for automatically threading a guidewire into a valve is provided according to a first aspect of the present application, including: a controller, a catheter driving mechanism and a guidewire driving mechanism that are in communicative connection with the controller, and a guidewire threading auxiliary assembly.

The catheter driving mechanism is provided with an angiographic valve body. The angiographic valve body is connected to a catheter disposed on the catheter driving mechanism. The guidewire driving mechanism includes a guidewire moving mechanism and a guidewire delivery mechanism mounted on the guidewire moving mechanism. The guidewire threading auxiliary assembly is mounted on a side of the guidewire driving mechanism close to the catheter driving mechanism.

In a working state, the guidewire driving mechanism is configured to clamp the guidewire, and the guidewire is partially located in the guidewire threading auxiliary assembly.

The controller is configured to obtain a guidewire threading instruction, control, based on the guidewire threading instruction, the guidewire moving mechanism to move towards the catheter driving mechanism until a part of the guidewire threading auxiliary assembly enters the angiographic valve body, control the guidewire moving mechanism to stop moving, and control the guidewire delivery mechanism to advance the guidewire into the catheter.

A fully-automatic angiographic robot is provided according to a second aspect of the present application, including the aforementioned device for automatically threading the guidewire into the valve, a front detection assembly in communicative connection with the controller, an angiographic injection device, and a guidewire threading valve body of the guidewire threading auxiliary assembly.

The angiographic injection device is connected to a side portion of the angiographic valve body.

Before obtaining the guidewire threading instruction, the controller is further configured to receive an angiographic instruction, and activate, based on the angiographic instruction, the guidewire driving mechanism to withdraw the guidewire from the angiographic valve body.

The front detection assembly is configured to detect the guidewire in real time, generate a second operation signal when the guidewire is withdrawn from the angiographic valve body, and send the second operation signal to the controller.

The controller is configured to control, based on the second operation signal, the guidewire driving mechanism to stop moving. The angiographic injection device is configured to inject a contrast agent into the angiographic valve body.

The guidewire threading valve body is disposed on the guidewire driving mechanism.

### BENEFICIAL EFFECTS

A device for automatically threading a guidewire into a valve and a fully-automatic angiographic robot are provided according to the present application. The device for automatically threading the guidewire into the valve includes a controller, a catheter driving mechanism and a guidewire driving mechanism that are in communicative connection with the controller, and a guidewire threading auxiliary assembly. The catheter driving mechanism is provided with an angiographic valve body. The angiographic valve body is connected to a catheter disposed on the catheter driving mechanism. The guidewire driving mechanism includes a guidewire moving mechanism and a guidewire delivery mechanism mounted on the guidewire moving mechanism. The guidewire threading auxiliary assembly is mounted on a side of the guidewire driving mechanism close to the catheter driving mechanism. In a working state, the guidewire is clamped by the guidewire driving mechanism and partially located in the guidewire threading auxiliary assembly. The controller is configured to obtain a guidewire threading instruction, control, based on the guidewire threading instruction, the guidewire moving mechanism to move towards the catheter driving mechanism until a part of the guidewire threading auxiliary assembly enters the angiographic valve body, control the guidewire moving mechanism to stop moving, and control the guidewire delivery mechanism to advance the guidewire into the catheter. This eliminates the need for the operators to manually monitor and thread the guidewire, significantly improving the efficiency of the angiographic procedure. Moreover, the fully-automatic angiographic robot is further provided with a coiled storage tube mounted on the guidewire driving mechanism, which prevents the guidewire from being exposed outside during the angiographic procedure, enhancing the safety of the procedure. Additionally, the fully-automatic angiographic robot is further provided with a first heparinized-saline-solution injection device and a second heparinized-saline-solution injection device for injecting a heparinized saline solution into the coiled storage tube and a guidewire channel in the guidewire threading valve body, preventing blood adhering to the guidewire surface from forming clots, thereby further improving the safety of the angiographic procedure.

### BRIEF DESCRIPTION OF THE DRAWINGS

FIG. 1 is a schematic structural view of a device for automatically threading a guidewire into a valve according to an embodiment of the present application;
FIG. 2 is a schematic structural view showing the internal structure of the device for automatically threading the guidewire into the valve according to an embodiment of the present application;
FIG. 3 is a schematic structural view of a fully-automatic angiographic robot according to an embodiment of the present application;
FIG. 4 is a schematic structural view of a guidewire threading valve body according to an embodiment of the present application; and
FIG. 5 is a schematic structural view of a first gear and a second gear according to an embodiment of the present application.

Reference numerals in the drawings are as follows:

| | | | |
|---|---|---|---|
| 1 | catheter driving mechanism; | 2 | guidewire driving mechanism; |
| 3 | guidewire threading auxiliary assembly; | 11 | angiographic valve body; |
| 12 | catheter; | 21 | guidewire moving mechanism; |
| 211 | guidewire delivery mechanism; | 31 | guidewire threading valve body; |
| 311 | guidewire channel; | 312 | heparinized-saline-solution injection port; |
| 313 | sealing ring; | 32 | guide member; |
| 321 | elongated tube; | 322 | guide connector; |
| 4 | front detection assembly; | 5 | angiographic injection device; |
| 6 | coiled storage tube; | 7 | rear detection assembly; |
| 8 | first gear; | 9 | second gear. |

Objects, functional characteristics, and advantages of the present application are further described with reference to the drawings in conjunction with the embodiments.

### DETAILED DESCRIPTION OF THE EMBODIMENTS

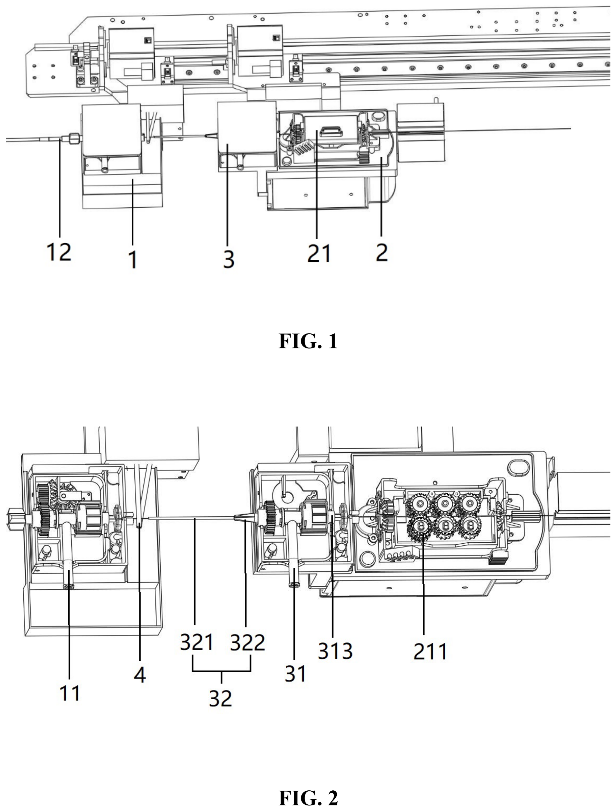

Referring to FIG. 1 and FIG. 2, a device for automatically threading a guidewire into a valve is provided according to an embodiment, including: a controller (not shown in the figures), a catheter driving mechanism 1 and a guidewire driving mechanism 2 that are in communicative connection with the controller, and a guidewire threading auxiliary assembly 3.

The catheter driving mechanism 1 is provided with an angiographic valve body 11. The angiographic valve body 11 is connected to a catheter 12 disposed on the catheter driving mechanism 1. The guidewire driving mechanism 2 includes a guidewire moving mechanism 21 and a guidewire delivery mechanism 211 mounted on the guidewire moving mechanism 21. The guidewire threading auxiliary assembly 3 is mounted on a side of the guidewire driving mechanism 2 close to the catheter driving mechanism 1.

In a working state, the guidewire driving mechanism 2 is configured to clamp the guidewire, and the guidewire is partially located in the guidewire threading auxiliary assembly 3.

The controller is configured to obtain a guidewire threading instruction, control, based on the guidewire threading instruction, the guidewire moving mechanism 21 to move towards the catheter driving mechanism 1 until a part of the guidewire threading auxiliary assembly 3 enters the angiographic valve body 11, control the guidewire moving mechanism 21 to stop moving, and control the guidewire delivery mechanism 211 to advance the guidewire into the catheter 12.

In this embodiment, the controller maintains communicative connection with the catheter driving mechanism 1 and the guidewire driving mechanism 2. The catheter driving mechanism 1, the guidewire driving mechanism 2, and the guidewire threading auxiliary assembly 3 are movable in a specified manner under the control of the controller. Preferably, the catheter driving mechanism 1, the guidewire driving mechanism 2, and the guidewire threading auxiliary assembly 3 always move along a same straight line to ensure linear movement of the guidewire.

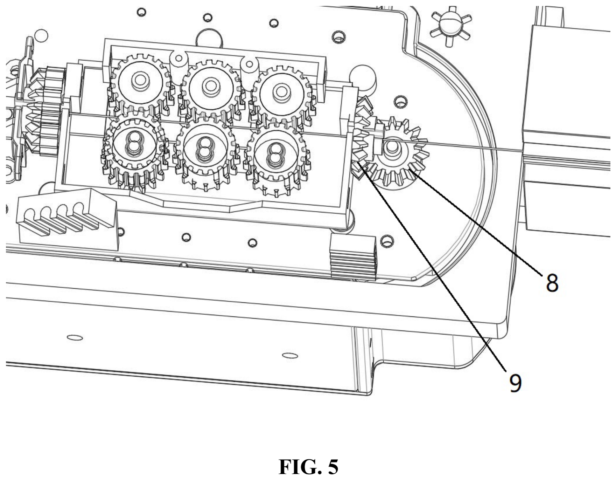

The catheter driving mechanism 1 is provided with an angiographic valve body 11, which includes but is not limited to a T-type valve, a Y-type valve, or a multi-port valve body. The angiographic valve body 11 allows passage of a guidewire and has a cavity with a volume for injection of a heparinized saline solution. It is understandable that in the present application, the angiographic valve body may also support functions such as heparin infusion, which is not limited herein. Furthermore, the angiographic valve body 11 is connected to the catheter 12 disposed on the catheter driving mechanism 1. The guidewire delivered from the guidewire driving mechanism 2 has to pass through the angiographic valve body 11 before entering the catheter 12. The guidewire driving mechanism 2 includes a guidewire moving mechanism 21, on which a guidewire delivery mechanism 211 is mounted to control the advancement and retraction of the guidewire. In this embodiment, the guidewire delivery mechanism 211 adopts several symmetrically distributed rollers, which clamp the guidewire. The rotation of the rollers enables the advancement and retraction of the guidewire through friction between the rollers and the guidewire. Referring to FIG. 5, preferably, a first gear 8 of the guidewire delivery mechanism 211 is integrally fixed to the guidewire delivery mechanism 211. When the first gear 8 rotates, it can drive the first gear 8 and the guidewire delivery mechanism 211 to rotate as a whole, thereby driving the entire guidewire to rotate.

Moreover, the guidewire threading auxiliary assembly 3 is mounted on the side of the guidewire driving mechanism 2 close to the catheter driving mechanism 1 to assist in delivering and holding the guidewire. In a working state, the guidewire is clamped by the guidewire driving mechanism 2 and partially enters the guidewire threading auxiliary assembly 3. When the guidewire is delivered, it is continuously advanced from the guidewire driving mechanism 2 into the guidewire threading auxiliary assembly 3. When the controller obtains a guidewire threading instruction, it controls the guidewire moving mechanism 21 to move towards the catheter driving mechanism 1 based on the guidewire threading instruction, until a part of the guidewire threading auxiliary assembly 3 enters the angiographic valve body 11. Then, the controller controls the guidewire moving mechanism 21 to stop moving. The delivery mechanism 211 advances the guidewire a certain distance into the catheter 12. This distance may be determined by the operator based on the depth of the catheter inserted into the human body, since the operator has inserted the catheter into a sheath tube and into the human blood vessel to a certain depth before the angiography operation. This improves the operation efficiency of the operator, eliminates the need for the doctor to manually monitor and thread the guidewire, thereby significantly improving the efficiency of threading the guidewire, and avoiding operational and judgmental errors during manual threading. In this embodiment, the sheath is a medical instrument used for insertion of the catheter into the human blood vessel.

Referring to FIG. 2, the guidewire threading auxiliary assembly 3 includes a guidewire threading valve body 31 and a guide member 32 disposed on the guidewire threading valve body 31. The guidewire threading valve body 31 is disposed on the guidewire driving mechanism 2. The guide member 32 includes an elongated tube 321 and a guide connector 322. The elongated tube 321 is configured to accommodate the guidewire, and the guide connector 322 connects the guidewire threading valve body 31 and the elongated tube 321.

In this embodiment, the guidewire threading auxiliary assembly 3 includes a guidewire threading valve body 31 and a guide member 32. The guide member 32 is disposed on the guidewire threading valve body 31, and the guidewire threading valve body 31 is disposed on the guidewire driving mechanism 2. Under the control of the controller, the guidewire threading valve body 31 can drive the guidewire threading valve body 31 to move. The guide member 32 includes an elongated tube 321 and a guide connector 322. The elongated tube 321 is configured to accommodate the guidewire, and the guide connector 322 connects the guidewire threading valve body 31 and the elongated tube 321, maintaining a stable connection between them. In this embodiment, a front part of the guidewire threading valve body 31 is connected with the guide member 32 via a Luer connector. The guidewire threading valve body 31 includes, but is not limited to, a T-type valve, a Y- type valve, a multi-port valve body, and a cavity that allows the guidewire to pass through and has a volume for injection of a heparinized saline solution.

Preferably, the guide member 32 is detachably mounted to the guidewire threading valve body 31.

In a specific embodiment, the guide member 32 is a guide needle.

In this embodiment, a guide needle serves as the guide member 32. The guide member 32 provides directional guidance and support to the guidewire passing through it, assisting in the delivery of the guidewire. The guide needle is disposed on the guidewire threading valve body 31. The operator has threaded the guidewire through the guidewire threading valve body 31 before the angiography operation. At this time, the guidewire partially enters the guide needle, and thus there is no need to thread the guidewire through the guidewire threading valve body 31 again when a subsequent angiography operation is required. The guide needle may have a cylindrical tubular shape and is made of rigid material. The cylindrical tubular shape may be a single piece or may be a combination of several parts, or the guide needle may have a shape matching a notch of the catheter 12.

Referring to FIG. 2, the device for automatically threading the guidewire into the valve further includes a front detection assembly 4 in communicative connection with the controller. The front detection assembly 4 is arranged at a side of the catheter driving mechanism 1 close to the guidewire driving mechanism 2. During the process of controlling the guidewire moving mechanism 21 to move towards the catheter driving mechanism 1 based on the guidewire threading instruction until a part of the guidewire threading auxiliary assembly 3 enters the angiographic valve body 11, the front detection assembly 4 is configured to detect a distal tip of the guidewire to determine whether the distal tip of the guidewire is located inside the guidewire threading auxiliary assembly 3.

If the distal tip of the guidewire is not detected, it is determined that the distal tip of the guidewire is located inside the guidewire threading auxiliary assembly 3. In this case, the front detection assembly 4 generates a first working signal and sends the first working signal to the controller. If the controller receives the first working signal, it continues controlling the guidewire moving mechanism 21 to move towards the catheter driving mechanism 1 until a part of the guidewire threading auxiliary assembly 3 enters the angiographic valve body 11.

If the distal tip of the guidewire is detected, it is determined that the distal tip of the guidewire is located outside the guidewire threading auxiliary assembly 3. In this case, the front detection assembly 4 generates a second working signal and sends the second working signal to the controller. If the controller receives the second working signal, it controls the guidewire moving mechanism 21 to stop moving towards the catheter driving mechanism 1, and controls the guidewire delivery mechanism 211 to retract the guidewire, until the controller receives the first working signal from the front detection assembly 4.

In this embodiment, a front detection assembly 4 is disposed at the side of the catheter driving mechanism 1 close to the guidewire driving mechanism 2 to prevent cases where the guidewire extends out of the guide needle but remains undetected due to bending of its tip portion. This front detection assembly 4 maintains communicative connection with the controller, which can control whether the front detection assembly 4 starts the detection. During the angiographic procedure, the guidewire moving mechanism 21 moves towards the catheter driving mechanism 1 under the control of the guidewire threading instruction until a part of the guidewire threading auxiliary assembly 3 enters the angiographic valve body 11. During this process, the front detection assembly 4 is configured to detect whether the distal tip of the guidewire is located inside the guidewire threading auxiliary assembly 3, that is, whether the guidewire extends out of the guide member 32. When the guidewire needs to be threaded into the valve, if the front detection assembly 4 detects the presence of the guidewire, the guidewire will be retracted under the control of the guidewire delivery mechanism 211 and will not extend out of the guide member 32. This ensures smart threading by the robot and avoids damage to the guidewire during the automatic threading process caused by the bending distal tip of the guidewire extending out of the guidewire threading auxiliary assembly 3. If the front detection assembly 4 does not detect the distal tip of the guidewire, it is determined that the distal tip of the guidewire is located inside the guidewire threading auxiliary assembly 3, indicating that the distal tip of the guidewire has entered the guide member 32 and the guidewire is ready for threading. At this time, the front detection assembly 4 generates a first working signal and sends it to the controller. If the controller receives the first working signal, it continues controlling the guidewire moving mechanism 21 to move towards the catheter driving mechanism 1 until a part of the guidewire threading auxiliary assembly 3 enters the angiographic valve body 11. If the distal tip of the guidewire is detected, it is determined that the distal tip of the guidewire is located outside the guidewire threading auxiliary assembly 3, indicating that the distal tip of the guidewire has left the guide member 32 and needs to be retracted back into the guide member 32 first. At this time, the front detection assembly 4 generates a second working signal and sends it to the controller. If the controller receives the second working signal, it controls the guidewire moving mechanism 21 to stop moving towards the catheter driving mechanism 1, and controls the guidewire delivery mechanism 211 to retract the guidewire, until the controller receives the first working signal from the front detection assembly 4. At this point, it indicates that the distal tip of the guidewire has entered the guide member 32, and guidewire threading is ready to start, thus achieving automatic guidewire threading. In this embodiment, the detection means of the front detection assembly 4 include, but are not limited to, a color sensor, laser, optical fiber, visual imaging, ultrasonics, a proximity sensor, and the like. Preferably, the front detection assembly 4 can accurately detect the presence or absence of the guidewire by using a laser or color sensor. Both laser and color sensors can directly detect the guidewire through a transparent sterile protective film or sterile housing.

In an embodiment, a sterile cover or a sterile housing is provided to cover the front detection assembly 4.

In this embodiment, a sterile cover or a sterile housing is provided to cover the sensor of the front detection assembly 4. The sterile component, such as a PE (Polyethylene) film, a transparent plastic housing, and a non-transparent plastic housing, can prevent the guidewire from being contaminated by the non-sterile sensor.

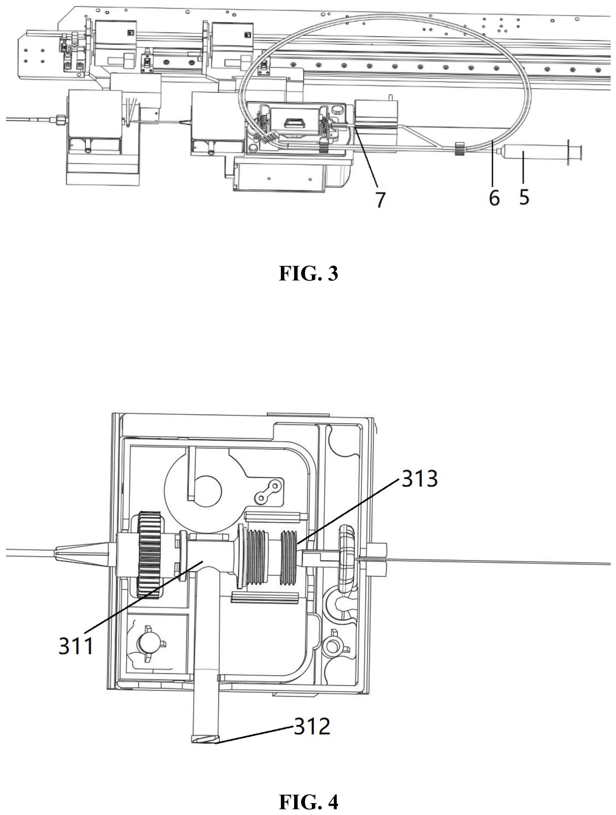

Referring to FIG. 3, a fully-automatic angiographic robot is further provided according to the present application, which includes the aforementioned device for automatically threading the guidewire into the valve, a front detection assembly 4 in communicative connection with the controller, an angiographic injection device 5, and a guidewire threading valve body 31 of the guidewire threading auxiliary assembly 3. The angiographic injection device 5 is connected to a side portion of the angiographic valve body 11. Before obtaining the guidewire threading instruction, the controller is further configured to receive an angiographic instruction, activate, based on the angiographic instruction, the guidewire driving mechanism 2 to withdraw the guidewire from the angiographic valve body 11. The front detection assembly 4 detects the guidewire in real time. When the guidewire is withdrawn from the angiographic valve body 11, the front detection assembly 4 generates a second working signal and sends it to the controller. Based on the second working signal, the controller controls the guidewire driving mechanism 2 to stop moving. The angiographic injection device 5 injects a contrast agent into the angiographic valve body 11. The guidewire threading valve body 31 is disposed on the guidewire driving mechanism 2.

In this embodiment, the fully-automatic angiographic robot includes the aforementioned device for automatically threading the guidewire into the valve and further includes the angiographic injection device 5 for injecting the contrast agent into the angiographic valve body 11. The angiographic injection device 5 is connected to the side portion of the angiographic valve body 11. Before obtaining the guidewire threading instruction, the controller is further configured to receive an angiographic instruction, based on which the controller controls the guidewire driving mechanism 2 to activate and withdraw the guidewire from the angiographic valve body 11. The front detection assembly 4 detects the guidewire in real time. When the guidewire is withdrawn from the angiographic valve body 11, the front detection assembly 4 generates a second working signal and sends it to the controller. Based on the second working signal, the controller controls the guidewire driving mechanism 2 to stop moving. During an angiography, the guidewire is withdrawn from the angiographic valve body 11. When the next angiography is required, the process of automatic guidewire threading is repeated to automatically thread the guidewire into the valve, eliminating the need for manual guidewire threading by the operator. This achieves automatic angiography, significantly reducing the workload of the operator and improving the safety of the angiographic procedure. In this embodiment, a coiled storage tube 6 is fixed to a sterile housing of a rear detection assembly 7.

Referring to FIG. 3, the fully-automatic angiographic robot further includes a coiled storage tube 6 and a first heparinized-saline-solution injection device. The coiled storage tube 6 is mounted on the guidewire driving mechanism 2.

A part of the guidewire is mounted inside the coiled storage tube 6. A first end of the coiled storage tube 6 is connected to the first heparinized-saline-solution injection device, which is configured to inject a heparinized saline solution into the coiled storage tube 6.

In this embodiment, the fully-automatic angiographic robot further includes a coiled storage tube 6 and a first heparinized-saline-solution injection device. The first heparinized-saline-solution injection device is not shown in the figures. The coiled storage tube 6 is mounted on the guidewire driving mechanism 2. A part of the guidewire is mounted inside the coiled storage tube 6. The first end of the coiled storage tube 6 is connected to the first heparinized-saline-solution injection device, which is configured to inject the heparinized saline solution into the coiled storage tube 6. The guidewire can be stored inside the coiled tube of the coiled storage tube 6, preventing it from falling to the ground and getting contaminated during the procedure, and reducing its likelihood of coming into contact with other contaminated areas. The heparinized saline solution injected into the coiled tube reduces the resistance of the guidewire inside the coiled tube, improving the stability of guidewire delivery, and lowering the resistance to guidewire rotation and delivery. Moreover, the blood adhering to the surface of the guidewire will be immersed in the heparinized saline solution inside the coiled storage tube 6, preventing the formation of blood clots, and significantly improving the safety of the procedure. In this embodiment, the coiled storage tube 6 may be a guidewire storage consumable provided by the guidewire supplier or other sterile cylindrical tubular materials.

Referring to FIG. 3, the fully-automatic angiographic robot further includes a rear detection assembly 7. The rear detection assembly 7 is disposed on the guidewire driving mechanism 2 and is configured to detect the guidewire inside the coiled storage tube 6, preventing the guidewire from departing from the coiled storage tube 6.

In this embodiment, the rear detection assembly 7 is disposed on the guidewire driving mechanism 2. During the procedure, the rear detection assembly 7 continuously detects the position of the guidewire within its detection range. If a proximal end of the guidewire exceeds the detection range of the rear detection assembly 7, the guidewire delivery mechanism is locked, and the delivery of the guidewire into the blood vessel is stopped, preventing the guidewire from departing from the coiled storage tube 6.

In a specific embodiment, the rear detection assembly 7 is a capacitive sensor.

In this embodiment, the rear detection assembly 7 is a capacitive sensor. Due to the minimal metal content in the guidewire, the arc-shaped, large-area capacitive sensor can precisely detect the presence or absence of the guidewire in the coiled tube at close range through a sterile film on the surface of the rear detection assembly 7, a plastic sterile housing, the coiled storage tube 6, and the heparinized saline solution inside the coiled tube.

Referring to FIG. 4, the guidewire threading valve body 31 includes a guidewire channel 311, a heparinized-saline-solution injection port 312 in communication with the guidewire channel 311, and a sealing ring 313 disposed in the guidewire channel 311 and located between the heparinized-saline-solution injection port 312 and the guidewire driving mechanism 2.

The fully-automatic angiographic robot further includes a second heparinized-saline-solution injection device.

The heparinized-saline-solution injection port 312 is connected to the second heparinized-saline-solution injection device, which is configured to inject a heparinized saline solution into the guidewire channel 311 through the heparinized-saline-solution injection port 312.

The sealing ring 313 is provided with a cross slit that allows the guidewire to pass through. When the guidewire is withdrawn from the angiographic valve body 11, the cross slit wipes off the blood and the heparinized saline solution from the guidewire.

In this embodiment, the guidewire channel 311 is configured to accommodate the guidewire. The heparinized-saline-solution injection port 312 connected to the guidewire channel 311 is configured to connect to an external second heparinized-saline-solution injection device. The heparinized saline solution injected from the second heparinized-saline-solution injection device flows into the guidewire channel 311 through the heparinized-saline-solution injection port 312. In this embodiment, the heparinized saline solution is intermittently replenished within the interior of the guidewire threading valve body 31 under the control of the second heparinized-saline-solution injection device. The second heparinized-saline-solution injection device may be a heparinized-saline-solution syringe or an external pressurized infusion or pump system. When the guidewire enters the guidewire threading valve body 31 filled with the heparinized saline solution, it is immersed in the heparinized saline solution, preventing the blood adhering to the guidewire from coagulating and forming blood clots, thereby improving the safety of the angiographic procedure. The sealing ring 313 is disposed in the guidewire channel 311 and located between the heparinized-saline-solution injection port 312 and the guidewire driving mechanism 2. The sealing ring 313 is provided with a cross slit through which the guidewire can pass through. When the guidewire is withdrawn from the angiographic valve body 11, the cross slit wipes off the blood and heparinized saline solution from the guidewire, thereby further preventing blood coagulation on the surface of the guidewire.

The above description shows only the preferred embodiment of the present application, and is not intended to limit the protection scope of the present application. Any equivalent structural or procedural modifications derived from the description and the drawings of the present application, no matter whether they are directly or indirectly applied in any other related technical field, shall be deemed to fall within the protection scope of the present application.

## Claims

1. A device for automatically threading a guidewire into a valve, comprising:
a controller;
a catheter driving mechanism and a guidewire driving mechanism that are in communicative connection with the controller; and
a guidewire threading auxiliary assembly, wherein
the catheter driving mechanism is provided with an angiographic valve body, and the angiographic valve body is connected to a catheter disposed on the catheter driving mechanism;
the guidewire driving mechanism comprises a guidewire moving mechanism and a guidewire delivery mechanism mounted on the guidewire moving mechanism, and the guidewire threading auxiliary assembly is mounted on a side of the guidewire driving mechanism close to the catheter driving mechanism;
in a working state, the guidewire driving mechanism is configured to clamp the guidewire, and the guidewire is partially located in the guidewire threading auxiliary assembly; and
the controller is configured to obtain a guidewire threading instruction, control, based on the guidewire threading instruction, the guidewire moving mechanism to move towards the catheter driving mechanism until a part of the guidewire threading auxiliary assembly enters the angiographic valve body, control the guidewire moving mechanism to stop moving, and control the guidewire delivery mechanism to advance the guidewire into the catheter.

2. The device for automatically threading the guidewire into the valve according to claim 1, wherein the catheter driving mechanism, the guidewire driving mechanism, and the guidewire threading auxiliary assembly are configured to always move along a same straight line.

3. The device for automatically threading the guidewire into the valve according to claim 1, wherein the angiographic valve body is a T-type valve, a Y-type valve, or a multi-port valve body.

4. The device for automatically threading the guidewire into the valve according to claim 1, wherein the guidewire delivery mechanism comprises a plurality of rollers that are symmetrically distributed, and the rollers are configured to clamp the guidewire and rotate to advance and retract the guidewire through friction between the rollers and the guidewire.

5. The device for automatically threading the guidewire into the valve according to claim 1, wherein
the guidewire threading auxiliary assembly comprises a guidewire threading valve body and a guide member disposed on the guidewire threading valve body;
the guidewire threading valve body is disposed on the guidewire driving mechanism; and
the guide member comprises an elongated tube and a guide connector, the elongated tube is configured to accommodate the guidewire, and the guide connector is configured to connect the guidewire threading valve body and the elongated tube.

6. The device for automatically threading the guidewire into the valve according to claim 5, wherein a front part of the guidewire threading valve body is connected with the guide member through a Luer connector, and the guidewire threading valve body comprises any one of a T-type valve, a Y-type valve, a multi-port valve body, and a cavity that allows passage of the guidewire and has a volume for injection of a heparinized saline solution.

7. The device for automatically threading the guidewire into the valve according to claim 5, wherein the guide member is detachably mounted to the guidewire threading valve body.

8. The device for automatically threading the guidewire into the valve according to claim 5, wherein the guide member is a guide needle.

9. The device for automatically threading the guidewire into the valve according to claim 8, wherein the guide needle has a cylindrical tubular shape and is made of rigid material.

10. The device for automatically threading the guidewire into the valve according to claim 1, further comprising a front detection assembly in communicative connection with the controller, wherein
the front detection assembly is disposed at a side of the catheter driving mechanism close to the guidewire driving mechanism;
during a process of controlling the guidewire moving mechanism to move towards the catheter driving mechanism based on the guidewire threading instruction until a part of the guidewire threading auxiliary assembly enters the angiographic valve body, the front detection assembly is configured to detect a distal tip of the guidewire to determine whether the distal tip of the guidewire is located inside the guidewire threading auxiliary assembly, wherein
in a case that the distal tip of the guidewire is not detected, the distal tip of the guidewire is determined to be located inside the guidewire threading auxiliary assembly, the front detection assembly is configured to generate a first working signal and send the first working signal to the controller, and the controller is configured to, in case of receiving the first working signal, continue controlling the guidewire moving mechanism to move towards the catheter driving mechanism until a part of the guidewire threading auxiliary assembly enters the angiographic valve body; and
in a case that the distal tip of the guidewire is detected, the distal tip of the guidewire is determined to be located outside the guidewire threading auxiliary assembly, the front detection assembly is configured to generate a second working signal and sends the second working signal to the controller, and the controller is configured to, in case of receiving the second working signal, control the guidewire moving mechanism to stop moving towards the catheter driving mechanism, and control the guidewire delivery mechanism to retract the guidewire until the controller receives the first working signal sent by the front detection assembly.

11. The device for automatically threading the guidewire into the valve according to claim 10, wherein the front detection assembly is configured for detection through any one of a color sensor, laser, optical fiber, visual imaging, ultrasonics, and a proximity sensor.

12. The device for automatically threading the guidewire into the valve according to claim 10, wherein a sterile cover or a sterile housing is provided to cover the front detection assembly.

13. A fully-automatic angiographic robot, comprising:
the device for automatically threading the guidewire into the valve according to any one of claims 1 to 12;
a front detection assembly in communicative connection with the controller;
an angiographic injection device; and
a guidewire threading valve body of the guidewire threading auxiliary assembly, wherein
the angiographic injection device is connected to a side portion of the angiographic valve body;
the controller is further configured to, before obtaining the guidewire threading instruction, receive an angiographic instruction and activate the guidewire driving mechanism based on the angiographic instruction, to withdraw the guidewire from the angiographic valve body;
the front detection assembly is configured to detect the guidewire in real time, generate a second working signal when the guidewire is withdrawn from the angiographic valve body, and send the second working signal to the controller;
the controller is configured to control, based on the second working signal, the guidewire driving mechanism to stop moving;
the angiographic injection device is configured to inject a contrast agent into the angiographic valve body; and
the guidewire threading valve body is mounted on the guidewire driving mechanism.

14. The fully-automatic angiographic robot according to claim 13, further comprising a coiled storage tube and a first heparinized-saline-solution injection device, wherein
the coiled storage tube is mounted on the guidewire driving mechanism; and
a part of the guidewire is mounted inside the coiled storage tube, a first end of the coiled storage tube is connected to the first heparinized-saline-solution injection device, and the first heparinized-saline-solution injection device is configured to inject a heparinized saline solution into the coiled storage tube.

15. The fully-automatic angiographic robot according to claim 14, further comprising a rear detection assembly, wherein the rear detection assembly is disposed on the guidewire driving mechanism and is configured to detect the guidewire inside the coiled storage tube, to prevent the guidewire from departing from the coiled storage tube.

16. The fully-automatic angiographic robot according to claim 15, wherein the rear detection assembly is a capacitive sensor.

17. The fully-automatic angiographic robot according to claim 13, wherein
the guidewire threading valve body comprises a guidewire channel, a heparinized-saline-solution injection port in communication with the guidewire channel, and a sealing ring disposed in the guidewire channel and located between the heparinized-saline-solution injection port and the guidewire driving mechanism;
the fully-automatic angiography robot further comprises a second heparinized-saline-solution injection device;
the heparinized-saline-solution injection port is connected to the second heparinized-saline-solution injection device, and the second heparinized-saline-solution injection device is configured to inject a heparinized saline solution into the guidewire channel through the heparinized-saline-solution injection port; and
the sealing ring is provided with a cross slit that allows passage of the guidewire, and the cross slit is configured to wipe off blood and the heparinized saline solution from the guidewire when the guidewire is withdrawn from the angiographic valve body.
